# EUROPÄISCHE PATENTSCHRIFT

(11) **EP 2 335 777 B1**
(45) Veröffentlichungstag und Bekanntmachung des Hinweises auf die Patenterteilung: **25.03.2015**
(21) Anmeldenummer: 10194113.6
(22) Anmeldetag: 08.12.2010
(51) Int. Cl.: A61N 5/10

(54) **Applikatoreinrichtung für die Röntgenstrahlentherapie sowie Strahlentherapievorrichtung**
Application device for X-ray therapy and radiotherapy device
Dispositif d'applicateur pour la thérapie radiographique ainsi que dispositif de thérapie par rayonnement

(30) Priorität: 17.12.2009 DE 102009058581
(43) Veröffentlichungstag der Anmeldung: 22.06.2011
(73) Patentinhaber: Carl Zeiss Meditec AG, 07745 Jena (DE)
(72) Erfinder: Kleinwächter, Timo, 72525 Münsingen (DE); Weigand, Frank, 89522 Heidenheim (DE); Wenz, Frederik, 69120 Heidelberg (DE)
(74) Vertreter: Müller & Schubert

(56) Entgegenhaltungen:
- EP-A1- 0 259 989
- EP-A1- 1 529 554
- EP-A2- 1 826 778
- CA-A2- 1 097 437
- US-A- 4 266 139
- US-A- 4 327 293
- US-A- 5 039 867
- US-A- 5 242 372
- US-A- 6 080 992
- US-A1- 2003 209 677
- US-A1- 2007 040 127
- US-B1- 7 109 505

## Beschreibung

Die vorliegende Erfindung betrifft zunächst eine Applikatoreinrichtung für die Röntgenstrahlentherapie gemäß dem Oberbegriff von Patentanspruch 1. Darüber hinaus betrifft die Erfindung auch eine Strahlentherapievorrichtung.

Die vorliegende Erfindung liegt insbesondere auf dem Gebiet der Strahlentherapie, insbesondere im Zusammenhang mit der Bestrahlung von Tumoren und dergleichen.

Strahlentherapievorrichtungen bestehen in der Regel aus einer Strahlungsquelleneinrichtung, bei deren Betrieb Strahlung, beispielsweise Röntgenstrahlung, entsteht. Über eine Sondeneinrichtung oder ein Strahlungsquellenelement wird die erzeugte Strahlung an den zu bestrahlenden Ort geführt. Dazu werden in der Regel so genannte Applikatoreinrichtungen eingesetzt.

Eine Applikatoreinrichtung besteht beispielsweise aus einem Applikatorelement, das zur Aufnahme einer Sondeneinrichtung oder eines Strahlungsquellenelements einer Strahlentherapievorrichtung ausgebildet ist. Das bedeutet, dass das Sondenelement oder das Strahlungsquellenelement der Strahltherapievorrichtung in das Applikatorelement eingeführt, beispielsweise eingeschoben, wird. Derartige Applikatorelemente sind im Stand der Technik bereits bekannt.

Beispielsweise ist in der Patentanmeldung DE 10 2008 030 590 A1 sowie in dem U.S. Patent 7,109,505 B1 der Anmelderin eine Applikatoreinrichtung beschrieben, bei der das Applikatorelement über einen Grundkörper verfügt, der aus einer Reihe verschiedener Bereiche besteht. Ein erster Bereich wird durch den Fußbereich gebildet. Er dient zur Aufnahme mindestens eines Bauelements einer Strahlentherapievorrichtung, beispielsweise wenigstens eines Bauelements einer Sondeneinrichtung oder eines Strahlungsquellenelements, die einen Bestandteil der Strahlentherapievorrichtung darstellt. An den Fußbereich schließt sich ein weitgehend zylindrischer Führungsbereich an, der zur Aufnahme und Führung einer Sondeneinrichtung oder einer Strahlungsquelleneinrichtung dient. Zwischen Fußbereich und Führungsbereich ist ein Übergangsbereich vorgesehen. Schließlich verfügt der Applikator an seinem distalen Ende über einen Kopfbereich, in dem insbesondere die für eine Bestrahlung benötigte Strahlung freigesetzt wird. Beim Betrieb der Strahlentherapievorrichtung entsteht in der Sondeneinrichtung oder im Strahlungsquellenelement Strahlung, die zumindest im Führungsbereich der Applikatoreinrichtung freigesetzt wird. Mit der Applikatoreinrichtung kann erreicht werden, dass Körpergewebe direkt am Ort eines Tumors bestrahlt werden kann. Die bekannte Applikatoreinrichtung wird dazu eingesetzt, um eine Bestrahlung in eng begrenzten Körperbereichen, insbesondere in Kanälen zu ermöglichen. Applikatoreinrichtungen können aber auch dazu dienen, Oberflächen zu bestrahlen, beispielsweise die Haut oder die Oberflächen von Organen.

Der vorliegenden Erfindung liegt die Aufgabe zugrunde, eine Applikatoreinrichtung, eine Befestigungseinrichtung sowie eine Strahlentherapievorrichtung der eingangs genannten Art derart weiterzubilden, dass diese in besonderer Weise auch für die Bestrahlung von Oberflächen geeignet sind.

Diese Aufgabe wird erfindungsgemäß gelöst durch die Applikatoreinrichtung mit den Merkmalen gemäß dem unabhängigen Patentanspruch 1. Weitere Merkmale und Details der Erfindung ergeben sich aus den Unteransprüchen, der Beschreibung sowie den Zeichnungen. Dabei gelten Merkmale und Details, die im Zusammenhang mit der erfindungsgemäßen Applikatoreinrichtung genannt sind, selbstverständlich auch im Zusammenhang mit der erfindungsgemäßen Befestigungseinrichtung sowie der erfindungsgemäßen Strahlentherapievorrichtung, und jeweils entsprechend umgekehrt.

Gemäß dem ersten Aspekt der vorliegenden Erfindung wird eine Applikatoreinrichtung für die Strahlentherapie, insbesondere für die Röntgenstrahlentherapie, zur Bestrahlung von Oberflächen bereitgestellt, mit einem Applikatorelement zur Aufnahme einer Sondeneinrichtung oder eines Strahlungsquellenelements einer Strahlentherapievorrichtung. Die Applikatoreinrichtung ist erfindungsgemäß **dadurch gekennzeichnet, dass** das Applikatorelement zur Einstellung verschiedener Strahlcharakteristika wenigstens ein austauschbar am/im Applikatorelement angeordnetes Element zur Strahlbeeinflussung aufweist. Insbesondere ist die Applikatoreinrichtung erfindungsgemäß auch **dadurch gekennzeichnet, dass** das Element zur Strahlbeeinflussung als Linsenelement oder als Linsenelementkombination ausgebildet ist.

Die Bestrahlung von Oberflächen beinhaltet dabei insbesondere eine Bestrahlung bis in eine Tiefe von 1 cm oder von ungefähr 1 cm.

Grundlage der vorliegenden Erfindung bildet somit eine Applikatoreinrichtung, welche durch den Austausch wenigstens eines Elements zur Strahlbeeinflussung verschiedene Strahlcharakteristiken erlaubt und ermöglicht.

Im Stand der Technik war es bisher bekannt, verschiedene Strahlcharakteristiken, um unterschiedlich tief in ein Gewebe einzustrahlen, dadurch zu realisieren, dass verschiedene Applikatorelemente bereitgehalten wurden. Dies war nicht nur aufwändig sondern auch kostenintensiv. Nunmehr können die verschiedenen Strahlcharakteristiken dadurch realisiert werden, dass das wenigstens eine Element zur Strahlbeeinflussung ausgetauscht wird. Die Applikatoreinrichtung als solche kann aber auch weiterhin noch benutzt werden. Dazu ist das Element zur Strahlbeeinflussung insbesondere lösbar am/im Applikatorelement angeordnet oder ausgebildet.

Die erfindungsgemäße Applikatoreinrichtung eignet sich insbesondere zur Bestrahlung der Haut oder von Oberflächen von Organen, beispielsweise zur oberflächlichen Bestrahlung von Läsionen oder Tumoren.

Bei der Applikatoreinrichtung kann es sich insbesondere um einen Oberflächen-Applikator für die Radiotherapie handeln.

Grundsätzlich ist die Erfindung nicht auf bestimmte Typen von Elementen zur Strahlbeeinflussung, oder aber auf eine bestimmte Anzahl solcher Elemente beschränkt. Bevorzugt kann wenigstens ein Element zur Strahlbeeinflussung als Linsenelement oder als Linsenelementkombination ausgebildet sein. Das Linsenelement kann einteilig oder mehrteilig ausgebildet sein. Mehrere Linsenelemente können zu einer Linsenelementkombination zusammengefasst sein. Bevorzugt ist vorgesehen, dass die Applikatoreinrichtung mit wenigstens einer auswechselbaren Linse zur Veränderung der Strahlcharakteristik ausgerüstet ist.

Nachfolgend werden einige bevorzugte Ausführungsbeispiele zur Ausgestaltung des Linsenelements oder der Linsenelementkombination beschrieben.

Bevorzugt kann das Linsenelement oder die Linsenelementkombination als Element oder Elementkombination mit einer unterschiedlichen Massenverteilung in einer Ebene quer zur Ausbreitungsrichtung der Röntgenstrahlung ausgebildet sein.

Bevorzugt kann die Massenverteilung des Linsenelements oder der Linsenelementkombination an die Abstrahlcharakteristik einer Strahlungsquelleneinrichtung angepasst sein oder anpassbar sein.

Die Massenverteilung ist eine maßgebliche Größe zur Beeinflussung der Röntgenstrahlung. Durch die Masse des Elements im Strahlungsweg der Röntgenstrahlung wird die Strahlung abgeschwächt, wobei eine größere Masse eine größere Abschwächung bewirkt. Die Massenverteilung kann dabei sowohl durch die Form wie auch durch die Wahl der Materialien des Linsenelements oder der Linsenelementkombination variiert werden.

Bevorzugt kann das Linsenelement oder die Linsenelementkombination zur Erzeugung einer Abstrahlcharakteristik der Applikatoreinrichtung unabhängig von der Art der Strahlungsquelleneinrichtung ausgebildet sein. Beispielsweise kann gezielt eine flache oder kugelförmige Abstrahlcharakteristik erzeugt werden, unabhängig davon, ob eine kugelförmige Strahlungsquelle oder eine gerichtete Strahlungsquelle, wie beispielsweise ein Linearbeschleuniger, verwendet wird.

Beispielsweise kann das Linsenelement oder die Linsenelementkombination und/oder das Applikatorelement - dieses zumindest bereichsweise - eine runde Form, oder eine viereckige Form, oder eine achteckige Form, oder eine an einen zu behandelnden Tumor angepasste Form, aufweisen.

Beispielsweise kann das Linsenelement oder die Linsenelementkombination aus einem oder wenigstens zwei unterschiedlichen Materialien bestehen.

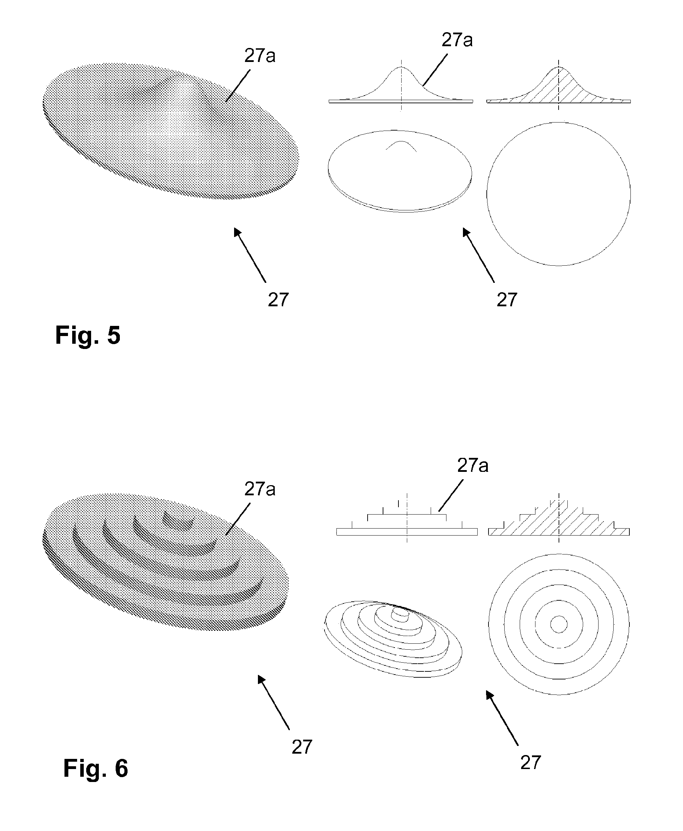

Bevorzugt kann das Linsenelement oder die Linsenelementkombination wenigstens eine positiv gekrümmte Oberfläche und/oder wenigstens eine neutrale Oberfläche und/oder wenigstens eine negativ gekrümmte Oberfläche aufweisen. Beispielsweise kann vorgesehen sein, dass die beiden Oberflächen gleich ausgebildet sind. Es ist jedoch auch möglich, dass die Oberflächen unterschiedlich ausgebildet sind. Positiv gekrümmte Oberflächen können beispielsweise konvexe Oberflächen, Oberflächen mit stetiger Kontur, Oberflächen mit einer Pyramidenstruktur, etwa einer Stufenpyramidenstruktur, und dergleichen sein. Neutrale Oberflächen gemäss der Erfindung sind insbesondere glatte, ebene Oberflächen. Negativ gekrümmte Oberflächen können beispielsweise konkave Oberflächen und dergleichen sein. Verschiedene Beispiele für unterschiedliche Linsenelemente sind im Zusammenhang mit den Figuren 5 bis 19 beschrieben, auf deren Offenbarungsgehalt auch an dieser Stelle voll inhaltlich Bezug genommen und verwiesen wird. Dabei können die gezeigten Ausführungsbeispiele sowohl jeweils einzeln, aber auch in jeder beliebigen Kombination realisiert sein.

Weiterhin ist die Erfindung auch nicht auf bestimmte Ausführungsformen der Applikatoreinrichtung beschränkt. Beispielsweise kann die Applikatoreinrichtung ein Applikatorelement aufweisen, das zunächst über einen Fußbereich verfügt, über welchen das Applikatorelement an einer Strahlungsquelleneinrichtung befestigt werden kann. So können die Strahlungsquelleneinrichtung und die Applikatoreinrichtung gemeinsam bewegt werden. An den Fußbereich kann sich beispielsweise ein Führungsbereich, insbesondere ein zylindrischer Führungsbereich anschließen. Der Führungsbereich weist insbesondere einen Aufnahmeraum auf, in welchen ein Sondenelement oder ein Strahlungsquellenelement einer Strahlentherapievorrichtung eingeführt werden kann. Ebenso kann auch der Fußbereich einen entsprechenden Aufnahmeraum aufweisen.

Insbesondere kann vorgesehen sein, dass das Applikatorelement zumindest bereichsweise zylindrisch ausgebildet ist.

Das Applikatorelement kann ein erstes, freies Ende aufweisen, das insbesondere einem zweiten Ende gegenüberliegt. Das zweite Ende dient beispielsweise zur Befestigung der Applikatoreinrichtung an der Strahlungsquelleneinrichtung. Das erste, freie Ende dient beispielsweise dazu, um auf der zu bestrahlenden Oberfläche platziert zu werden. Bevorzugt kann in einem solchen Fall vorgesehen sein, dass das Element zur Strahlbeeinflussung im Bereich des ersten Endes des Applikatorelements vorgesehen ist.

Im vorgenannten Fall erfolgt die Bestrahlung, beziehungsweise die Abgabe der Strahlung, insbesondere über das erste, freie Ende des Applikatorelements. Das heißt, die Abgabe der Strahlung erfolgt nach vorne. Selbstverständlich ist die Erfindung nicht auf dieses Ausführungsbeispiel beschränkt. Beispielsweise kann auch vorgesehen sein, dass die Abgabe der Strahlung in anderen Bereichen des Applikatorelements erfolgt. Beispielsweise kann eine Bestrahlung zur Seite realisiert werden. Natürlich ist auch denkbar, dass die Abgabe der Strahlung nicht nur in einem Bereich, sondern in mehreren verschiedenen Bereichen des Applikatorelements erfolgt.

Bei bisher bekannten Applikatoreinrichtungen, die zur Bestrahlung von Oberflächen genutzt werden, bestand das Problem, dass die Applikatoreinrichtungen in der Regel nur sehr aufwändig an der zu bestrahlenden Fläche befestigt werden konnten. Auch dieses Problem kann mit der vorliegenden Erfindung beseitigt werden.

Gemäß einem zweiten Aspekt der Erfindung, oder aber als weitere bevorzugte Ausführungsform der erfindungsgemäßen Applikatoreinrichtung, wird eine Befestigungseinrichtung zur Befestigen einer Applikatoreinrichtung für die Strahlentherapie, insbesondere für die Röntgenstrahlentherapie, insbesondere zur Bestrahlung von Oberflächen, auf einer zu behandelnden Oberfläche bereitgestellt, die **dadurch gekennzeichnet ist, dass** die Befestigungseinrichtung eine Aufnahmeöffnung zur Aufnahme zumindest eines Bereichs der Applikatoreinrichtung aufweist.

Dadurch kann die Applikatoreinrichtung, beispielsweise über deren Applikatorelement, in welchem eine Sondeneinrichtung oder eine Strahlungsquellenelement einer Strahlentherapievorrichtung eingeführt ist, in die Aufnahmeöffnung der Befestigungseinrichtung eingesetzt werden. Die Befestigungseinrichtung wiederum kann an der zu bestrahlenden Oberfläche befestigt werden.

Die Befestigungseinrichtung weist bevorzugt einen Grundkörper auf, der an der zu behandelnden Oberfläche angeordnet beziehungsweise befestigt wird. Weiterhin weist die Befestigungseinrichtung die Aufnahmeöffnung auf, die insbesondere in dem Grundkörper ausgebildet ist und diesen beispielsweise durchdringt. In einem solchen Fall dienen die Seitenwandungen der Aufnahmeöffnung, welche den Grundkörper durchdringt, zumindest bereichsweise zur Abstützung und Fixierung der Applikatoreinrichtung in der Befestigungseinrichtung.

Dabei ist die Erfindung weder auf bestimmte Ausführungsformen der Befestigungseinrichtung, noch auf bestimmte Möglichkeiten der Befestigung beschränkt. Einige vorteilhafte, jedoch nicht ausschließliche Beispiele werden im weiteren Verlauf der Beschreibung näher erläutert.

Die Befestigungseinrichtung ist erfindungsgemäß insbesondere auch **dadurch gekennzeichnet, dass** diese als Befestigungsring ausgebildet ist.

Vorzugsweise weist die Aufnahmeöffnung eine Innenkontur auf, die der Außenkontur des aufzunehmenden Bereichs der Applikatoreinrichtung entspricht. Dadurch, dass die Aufnahmeöffnung der Befestigungseinrichtung eine Innenkontur aufweist, die der Außenkontur des aufzunehmenden Bereichs der Applikatoreinrichtung entspricht, kann sichergestellt werden, dass die Applikatoreinrichtung einen festen, insbesondere verrutschsicheren Halt in der Befestigungseinrichtung hat. Dabei ist die Erfindung nicht auf bestimmte Ausgestaltungsformen der Aufnahmeöffnung beschränkt.

Beispielsweise kann vorgesehen sein, dass die Befestigungseinrichtung eine runde Aufnahmeöffnung aufweist, und dass die runde Aufnahmeöffnung einen Durchmesser aufweist, der dem Außendurchmesser einer aufzunehmenden zylindrischen Applikatoreinrichtung entspricht.

Natürlich sind auch andere Ausführungsformen denkbar. So kann die Aufnahmeöffnung beispielsweise auch viereckig, vorzugsweise quadratisch, ausgebildet sein, während der aufzunehmende Bereich der Applikatoreinrichtung einen runden, beispielsweise zylindrischen, Querschnitt aufweist. Die Seitenlängen des Vierecks können dann vorzugsweise dem Durchmesser des runden Abschnitts entsprechen, so dass die Applikatoreinrichtung sicher in der Aufnahmeöffnung und damit in der Befestigungseinrichtung befestigt und fixiert werden kann. Natürlich sind auch andere Geometrien denkbar. Wichtig ist lediglich, dass die Applikatoreinrichtung in die Aufnahmeöffnung der Befestigungseinrichtung eingeführt und in dieser gehalten werden kann.

Wenn die Bestrahlung beziehungsweise die Abgabe nach vorne, das heißt über das erste, freie ende des Applikatorelements erfolgen soll, ist die Befestigungseinrichtung vorzugsweise in der vorstehend genannten Weise, beispielsweise als Befestigungsring, ausgebildet. Wenn eine Bestrahlung beziehungsweise die Abgabe der Strahlung über andere Bereiche des Applikatorelements, beispielsweise über die Seite, erfolgen soll, kann die Befestigungseinrichtung in geeigneter Weise ausgebildet sein, die eine Abgabe der Strahlung zur Seite ermöglicht. Beispielsweise könnte die Befestigungseinrichtung in einem solchen Fall L-förmig ausgestaltet sein.

Die Befestigungseinrichtung wird auf der zu behandelnden Oberfläche befestigt. Dies kann auf unterschiedliche Weise erfolgen.

Beispielsweise kann vorgesehen sein, dass die Befestigungseinrichtung direkt, beispielsweise mit doppelseitigem Klebeband, auf der zu bestrahlenden Oberfläche befestigt und fixiert wird.

Bevorzugt kann vorgesehen sein, dass die Befestigungseinrichtung an wenigstens einer ihrer Außenseiten wenigstens eine nach außen abragende Befestigungslasche aufweist. Natürlich können auch mehrere solcher Befestigungslaschen vorgesehen sein. Bei Vorhandensein solcher Befestigungslaschen kann beispielsweise vorgesehen sein, dass diese mit normalem Klebeband auf der zu bestrahlenden Oberfläche befestigt und fixiert werden.

Bevorzugt kann wenigsten eine Befestigungslasche wenigstens eine Befestigungsöffnung aufweisen. Auf diese Weise wird es insbesondere ermöglicht, dass die Befestigungseinrichtung auch festgenäht werden kann. Dies kann beispielsweise bei der Bestrahlung von Organen von Vorteil sein.

Gemäß einem dritten Aspekt der Erfindung wird eine Strahlentherapievorrichtung, insbesondere zur Röntgbestrahlung, insbesondere zur Bestrahlung von Oberflächen, mit einer Strahlungsquelleneinrichtung bereitgestellt. Die Strahlentherapievorrichtung weist eine Applikatoreinrichtung zur Aufnahme einer Sondeneinrichtung oder eines Strahlungsquellenelements der Strahlungsquelleneinrichtung auf, wobei die Applikatoreinrichtung in der wie weiter oben beschriebenen erfindungsgemäßen Weise ausgebildet ist, so dass diesbezüglich auch auf die vorstehenden Ausführungen zur Applikatoreinrichtung vollinhaltlich Bezug genommen und verwiesen wird. Alternativ oder zusätzlich kann die Strahlentherapievorrichtung eine wie vorstehend beschriebene erfindungsgemäße Befestigungseinrichtung zur Befestigen einer Applikatoreinrichtung für die Strahlentherapie auf einer zu behandelnden Oberfläche aufweisen, so dass diesbezüglich auch auf die vorstehenden Ausführungen zur Befestigungseinrichtung vollinhaltlich Bezug genommen und verwiesen wird.

Die Strahlentherapievorrichtung kann insbesondere zur Bestrahlung von Oberflächen, beispielsweise zur Bestrahlung der Haut oder der Oberfläche eines Organs, eingesetzt werden.

Die Strahlentherapievorrichtung weist zunächst eine Strahlungsquelleneinrichtung auf, mittels derer die zur Bestrahlung erforderlichen Strahlungsdosen erzeugt werden. Insbesondre ist die Strahlungsquelleneinrichtung zur Erzeugung von Strahlen für die Radiotherapie ausgebildet. An der Strahlungsquelleneinrichtung wird die Applikatoreinrichtung angeordnet.

Die Erfindung wird nun anhand von Ausführungsbeispielen unter Bezugnahme auf die beiliegenden Zeichnungen näher erläutert. Es zeigen
- Figur 1: eine perspektivische Darstellung einzelner Bauteile der erfindungsgemäßen Strahlentherapievorrichtung mit erfindungsgemäßer Applikatoreinrichtung;
- Figur 2: eine Darstellung der Strahlentherapievorrichtung gemäß Figur 1 in zusammengebautem Zustand;
- Figur 3: eine Querschnittsansicht der erfindungsgemäßen Strahlentherapievorrichtung gemäß Figur 2;
- Figur 4: eine Darstellung einer Befestigungseinrichtung zur Befestigung einer Applikatoreinrichtung; und
- Figuren 5 bis 19: verschiedene Ausführungsbeispiele für Elemente zur Strahlbeeinflussung, die in einer Strahlentherapievorrichtung gemäß den Figuren 1 bis 3 zum Einsatz kommen können.

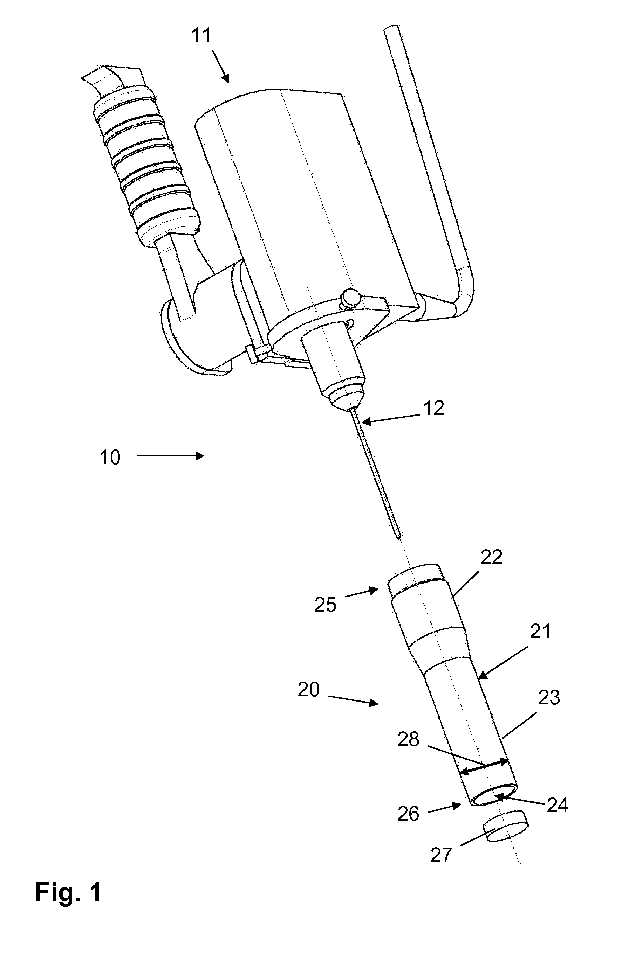

In den Figuren 1 bis 3 ist eine Strahlentherapievorrichtung 10 dargestellt. Die Strahlentherapievorrichtung 10 weist eine Strahlungsquelleneinrichtung 11 auf, die zur Erzeugung von Strahlung für die Radiotherapie ausgebildet ist. Zum Applizieren der erzeugten Strahlung ist ein Strahlungsquellenelement 12 vorgesehen. Die Strahlentherapievorrichtung 10 dient zur Röntgenstrahlentherapie.

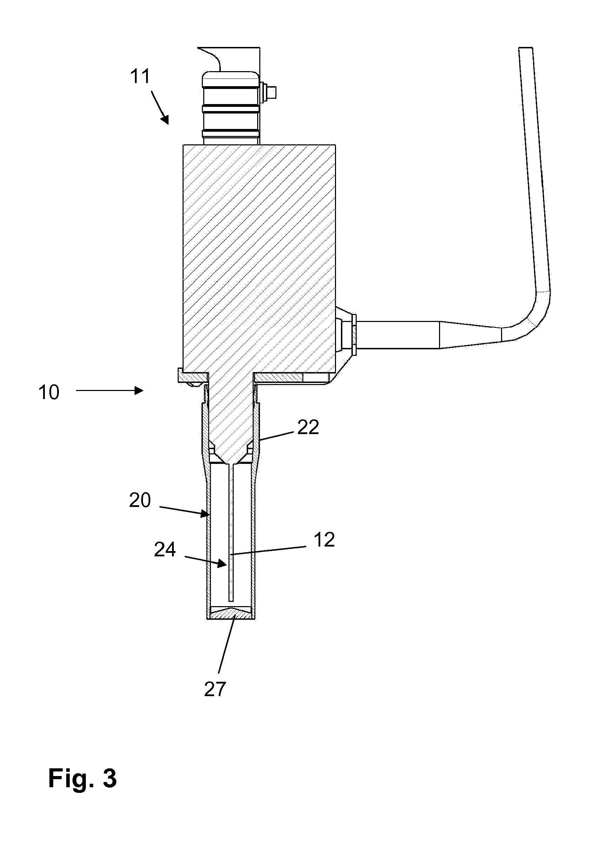

Wie in den Figuren 1 bis 3 weiter dargestellt ist, weist eine erfindungsgemäße Applikatoreinrichtung 20 ein zylindrisch ausgeformtes Applikatorelement 21 auf. Das Applikatorelement 21 besteht aus einem Fußbereich 22 und einem sich daran anschließenden Führungsbereich 23. Insbesondere im Führungsbereich 23 befindet sich im Bestrahlungsbetrieb das Strahlungsquellenelement 12 der Strahlungsquelleneinrichtung 11 der Strahlentherapievorrichtung 10, wie dies insbesondere auch in Figur 3 verdeutlicht ist. Dazu weist der Führungsbereich 23 des Applikatorelements 21 einen entsprechenden Aufnahmeraum 24 auf.

Das Applikatorelement 21 weist im Fußbereich 22 ein zweites Ende 25 auf, über welches das Applikatorelement 21, und damit die Applikatoreinrichtung 20, an der Strahlungsquelleneinrichtung 11 befestigbar ist. Bei dem dem zweiten Ende 25 gegenüberliegenden ersten Ende 26 des Applikatorelements 21 handelt es sich um das den Führungsbereich 23 des Applikatorelements 21 abschließende freie Ende des Applikatorelements 21, über welches die Strahlendosis beispielsweise auf die zu bestrahlende Oberfläche appliziert werden kann.

Um mit der Applikatoreinrichtung 20 verschiedene Strahlcharakteristiken erzeugen zu können, beispielsweise um unterschiedlich tief in ein zu bestrahlendes Gewebe einstrahlen zu können, weist die Applikatoreinrichtung 20 in dem in den Figuren 1 bis 3 gezeigten Ausführungsbeispiel ein Element 27 zur Strahlbeeinflussung auf. Das Element 27 zur Strahlbeeinflussung ist vorteilhaft als Linse 27 zur Veränderung der Strahlcharakteristik ausgebildet, Es ist bevorzugt auswechselbar, das heißt lösbar, an dem Applikatorelement 21 angeordnet, bevorzugt im Bereich von dessen ersten freien Ende 26.

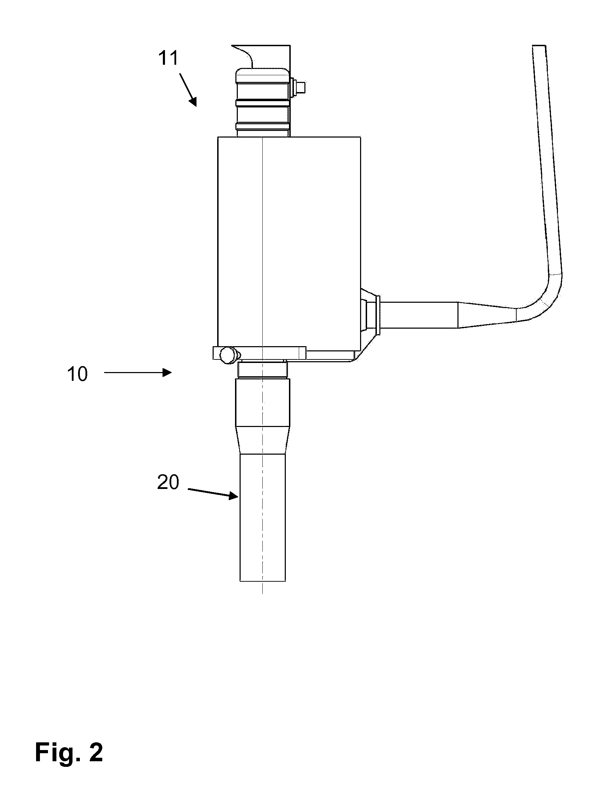

In Figur 2 ist die Strahlentherapievorrichtung 10 gemäß Figur 1 in einem Zustand gezeigt, in dem das Applikatoreinrichtung 20 und die Strahlentherapievorrichtung 10 zusammengebaut sind. Figur 3 zeigt eine Querschnittsansicht der in Figur 2 dargestellten zusammengebauten Strahlentherapievorrichtung 10.

In Figur 3 ist insbesondere dargestellt, wie die Applikatoreinrichtung 20 über ihren Fußbereich 22 an der Strahlungsquelleneinrichtung 11 befestigt ist. Die Strahlungsquelleneinrichtung 11 und die Applikatoreinrichtung 20 bilden Bestandteile der Strahlentherapievorrichtung 10. In der Strahlungsquelleneinrichtung 11 wird eine Strahlung, beispielsweise eine Strahlung für die Radiotherapie, erzeugt und über das Strahlungsquellenelement 12, das sich an die Strahlungsquelleneinrichtung 11 anschließt, und das sich im Aufnahmeraum 24 innerhalb des Applikatorelements 21 befindet, auf die zu bestrahlende Oberfläche appliziert.

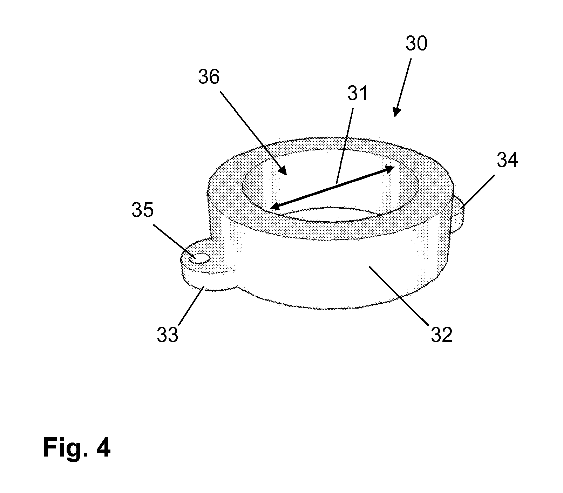

Insbesondere dann, wenn eine Oberfläche bestrahlt werden soll, gestaltet es sich oftmals als schwierig, die Applikatoreinrichtung 20 an der zu bestrahlenden Stelle zu befestigen. Um dieses Problem zu beseitigen, ist eine besondere Befestigungseinrichtung 30 vorgesehen, die im Zusammenhang mit Figur 4 nachfolgend näher beschrieben wird.

Die Befestigungseinrichtung 30 ist als Befestigungsring ausgebildet, über den die Applikatoreinrichtung 20 auf der zu behandelnden Oberfläche befestigt und fixiert werden kann. Der Befestigungsring 31 hat eine Aufnahmeöffnung 36 für das Applikatorelement 21, wobei die Aufnahmeöffnung 36 einen Innendurchmesser 31 aufweist, der dem Außendurchmesser 28 des Applikatorelements 21 im Bereich von dessen erstem Ende 26 entspricht, wie dies auch in den Figuren 1 und 3 verdeutlicht ist. Dadurch kann die Applikatoreinrichtung 20 in den Befestigungsring 30 eingesetzt und verrutschsicher in diesem gehalten werden.

Der Befestigungsring 30 wiederum kann auf der zu behandelnden Oberfläche, beispielsweise der Haut, befestigt und fixiert werden, etwa unter Verwendung von doppelseitigem Klebeband. Alternativ kann vorgesehen sein, dass der Befestigungsring 30 wenigstens eine von dessen Außenseite 32 nach außen abragende Befestigungslasche aufweist. In Figur 4 sind zur Verdeutlichung zwei solcher Befestigungslaschen 33, 34 dargestellt, wobei die Anzahl je nach Anwendungsfall variieren kann. In einem solchen Fall kann der Befestigungsring 30 über die Befestigungslaschen 33, 34 auch mit normalem Klebeband auf der zu behandelnden Oberfläche befestigt und fixiert werden. Befinden sich in den Befestigungslaschen 33, 34 Befestigungsöffnungen 35, kann der Befestigungsring 30 beispielsweise auch festgenäht werden. Dies kann beispielsweise bei der Bestrahlung von Organen von Vorteil sein.

Nachfolgend werden anhand der Figuren 5 bis 19 verschiedene Ausführungsbeispiele von Elementen 27 zur Strahlbeeinflussung dargestellt, die in einer wie vorstehend beschriebenen Applikatoreinrichtung 20 zum Einsatz kommen können. Bei den Elementen 27 handelt es sich um Linsenelemente. Ein Linsenelement im Sinne der Patentanmeldung ist dabei insbesondere ein Element mit einer unterschiedlichen Masseverteilung in einer Ebene quer zur Ausbreitungsrichtung der Röntgenstrahlung. Die Massenverteilung des Linsenelements 27 ist dabei idealerweise an eine Abstrahlcharakteristik der Strahlungsquelleneinrichtung 11 angepasst. Die Massenverteilung ist ein maßgebliche Größe zur Beeinflussung der Röntgenstrahlung. Durch die Masse des Linsenelements 27 im Strahlungsweg der Röntgenstrahlung wird die Strahlung abgeschwächt, wobei eine größere Masse eine größere Abschwächung bewirkt. Die Massenverteilung kann dabei sowohl durch die Form als auch durch die Wahl der Materialien des Linsenelements 27 variiert werden.

Auf diese Weise kann eine Abstrahlcharakteristik der Applikatoreinrichtung 20 unabhängig von der Art der Strahlungsquelleneinrichtung 11 angepasst werden. Nachfolgend werden verschiedene Ausführungsbeispiele für Linsenelemente 27 mit verschiedenen Massenverteilungen beschrieben, die für die in den Figuren 1 bis 3 dargestellte Applikatoreinrichtung 20 verwendet werden können.

Figur 5 zeigt ein Element 27 zur Strahlbeeinflussung in Form eines Linsenelements, das aus einem Material besteht und eine runde Form hat. Eine Oberfläche 27a des Linsenelements 27 weist dabei eine positive Krümmung in Form einer stetigen Kontur auf.

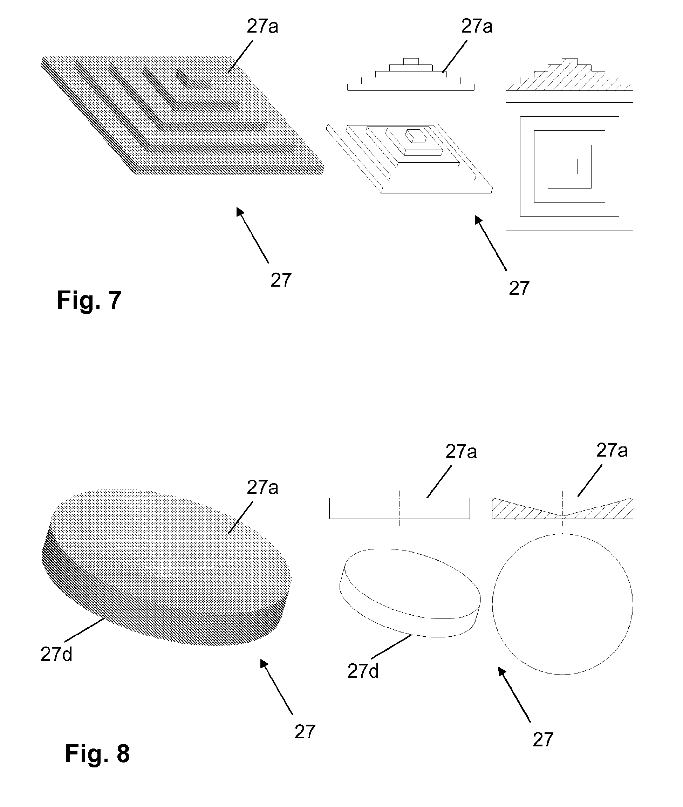

Figur 6 zeigt ein Element 27 zur Strahlbeeinflussung in Form eines Linsenelements, das aus einem Material besteht und eine runde Form hat. Eine Oberfläche 27a des Linsenelements 27 weist dabei eine positive Krümmung in Form einer Kontur in Form einer Stufenpyramide auf. Die einzelnen Stufen der Stufenpyramide könnten dabei zumindest teilweise auch aus unterschiedlichen Materialien bestehen. Figur 7 zeigt ein ähnliches Ausführungsbeispiel, nur dass das Linsenelement eine viereckige Form aufweist.

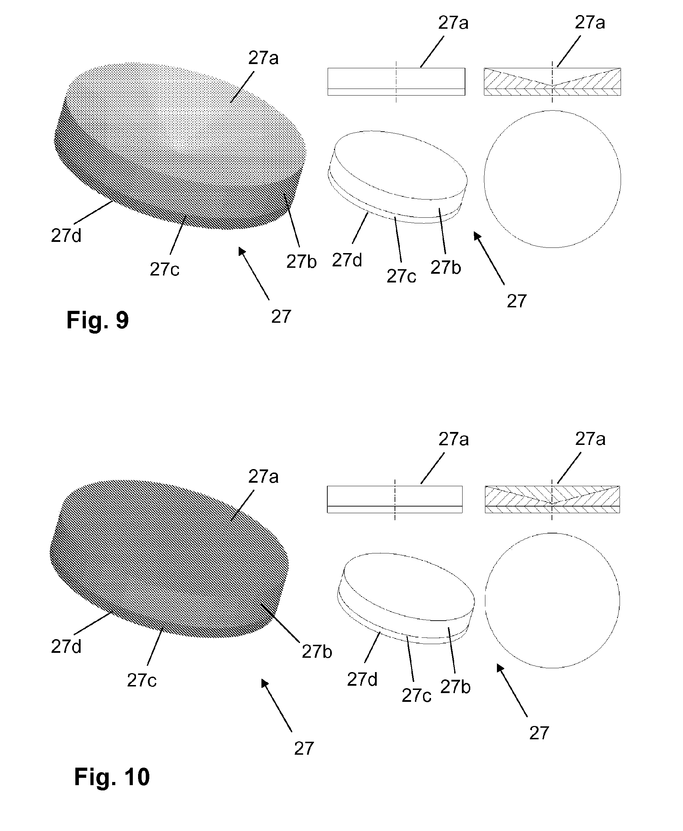

Figur 8 zeigt ein Element 27 zur Strahlbeeinflussung in Form eines Linsenelements, das aus einem Material besteht und eine runde Form hat. Eine Oberfläche 27a des Linsenelements 27 weist dabei eine negative Krümmung in Form einer konkaven Kontur auf. Die andere Oberfläche 27d weist eine neutrale, glatte Kontur auf. Bei dem gezeigten Element handelt es sich um ein Fokus-Element. Figur 9 zeigt ein ähnliches Ausführungsbeispiel, nur dass das Element aus zwei unterschiedlichen Materialien 27b, 27c besteht. Das in Figur 10 dargestellte Ausführungsbeispiel ist ähnlich wie das in Figur 9 dargestellte Ausführungsbeispiel, wobei die Oberfläche neutral in Form einer glatten Oberfläche ausgebildet ist. Dies kann beispielsweise durch eine Auffüllung erfolgen.

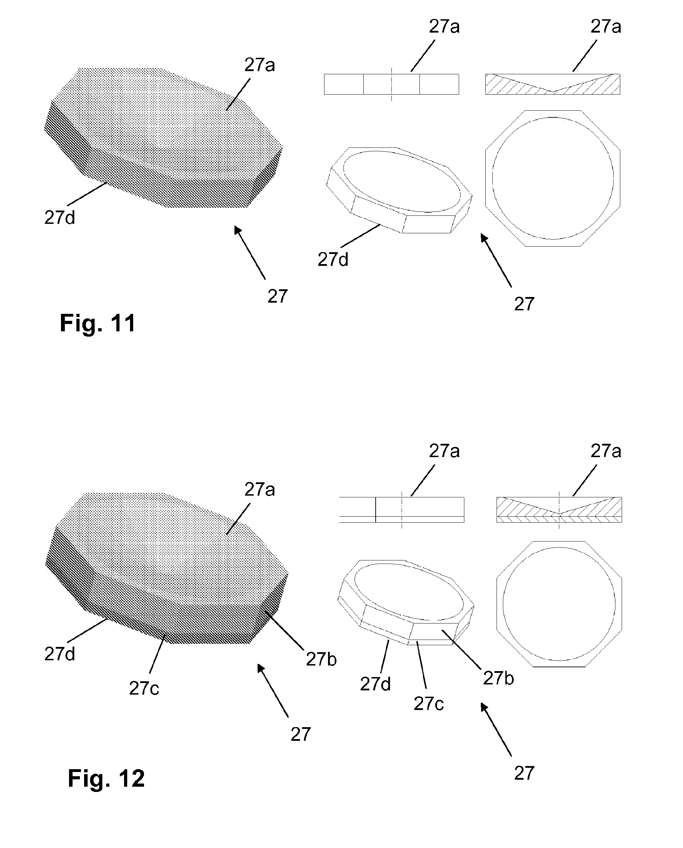

Figur 11 zeigt ein Element 27 zur Strahlbeeinflussung in Form eines Linsenelements, das aus einem Material besteht und eine achteckige Form hat. Eine Oberfläche 27a des Linsenelements 27 weist dabei eine negative Krümmung in Form einer konkaven Kontur auf. Die andere Oberfläche 27d weist eine neutrale, glatte Kontur auf. Bei dem gezeigten Element handelt es sich um ein Fokus-Element. Figur 12 zeigt ein ähnliches Ausführungsbeispiel, nur dass das Element aus zwei unterschiedlichen Materialien 27b, 27c besteht.

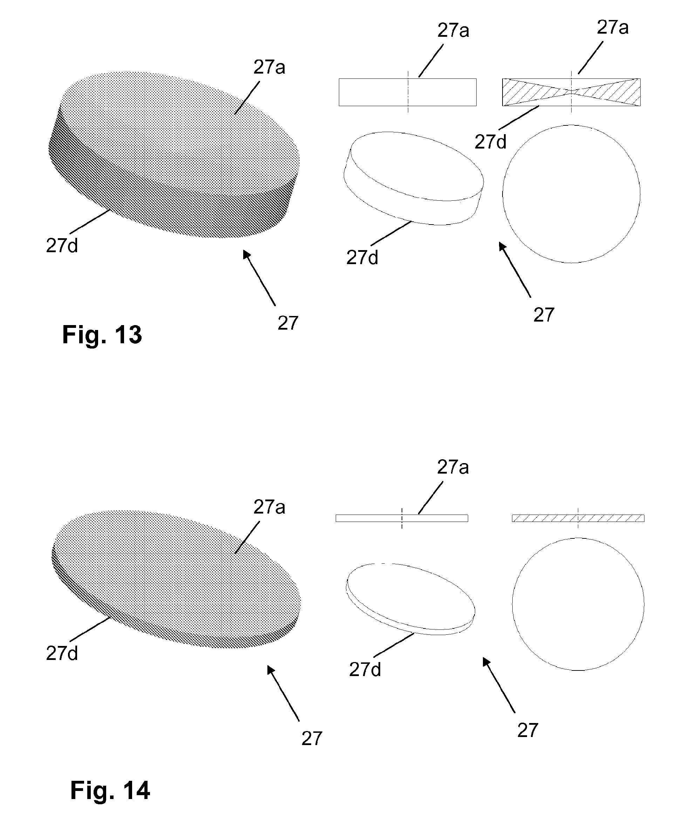

Figur 13 zeigt ein Element 27 zur Strahlbeeinflussung in Form eines Linsenelements, das aus einem Material besteht und eine runde Form hat. Beide Oberflächen 27a, 27d des Linsenelements 27 weisen dabei eine negative Krümmung in Form einer konkaven Kontur auf. Bei dem gezeigten Element handelt es sich um ein Fokus-Element.

Figur 14 zeigt ein Element 27 zur Strahlbeeinflussung in Form eines Linsenelements, das aus einem Material besteht und eine runde, flache Form hat. Beide Oberflächen 27a, 27d des Linsenelements 27 weisen dabei eine neutrale, glatte Kontur auf.

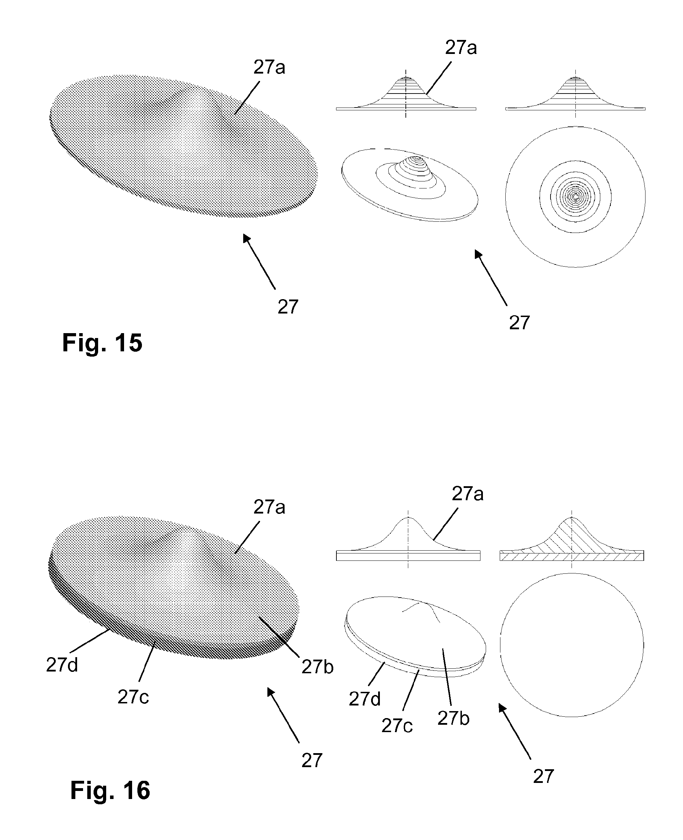

Figur 15 zeigt ein Element 27 zur Strahlbeeinflussung in Form eines Linsenelements, das eine runde Form hat. Eine Oberfläche 27a des Linsenelements 27 weist dabei eine positive Krümmung in Form einer stetigen Kontur auf. Das Element 27 besteht aus mehreren Materialien. Im dargestellten Ausführungsbeispiel besteht das Element 27 aus Materialien mit abnehmender Dichte.

Figur 16 zeigt ein Element 27 zur Strahlbeeinflussung in Form eines Linsenelements, das eine runde Form hat. Eine Oberfläche 27a des Linsenelements 27 weist dabei eine positive Krümmung in Form einer stetigen Kontur auf. Die andere Oberfläche 27d weist eine neutrale, glatte Kontur auf. Das Element 27 besteht aus mehreren Materialien 27b, 27c.

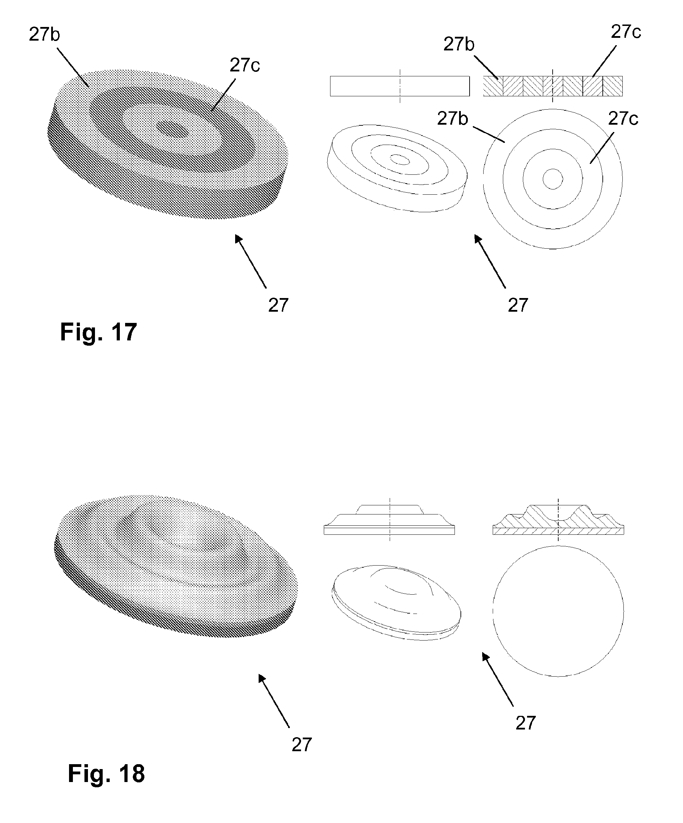

Figur 17 zeigt ein anderes Ausführungsbeispiel für ein Element 27 zur Strahlbeeinflussung in Form eines Linsenelements, das aus zwei unterschiedlichen Materialien 27b, 27c besteht. Dabei ist das Element 27 ringförmig ausgebildet.

Figur 18 zeigt noch ein weiteres Ausführungsbeispiel eines Elements 27 zur Strahlbeeinflussung in Form eines Linsenelements mit einer ungewöhnlichen Form.

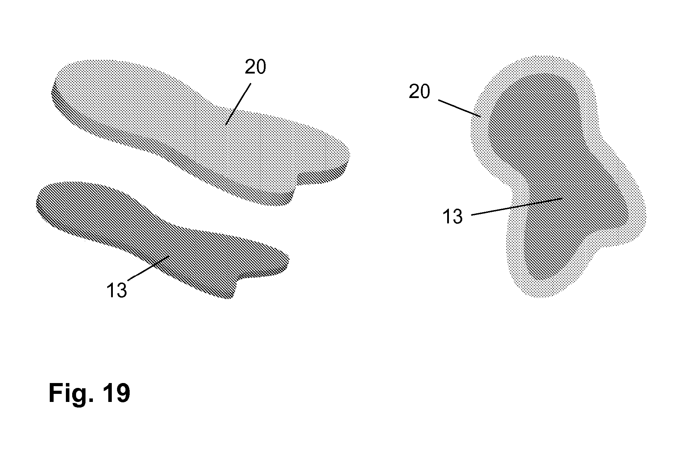

In Figur 19 schließlich ist ein Ausführungsbeispiel dargestellt, bei dem die Applikatoreinrichtung 20, alternativ oder zusätzlich das Element 27 zur Strahlbeeinflussung an die Form eines zu behandelnden Tumors 13 angepasst ist/sind.

Die in den Figuren dargestellten und vorstehend beschriebenen Ausführungsbeispiele eigenen sich insbesondere zur Bestrahlung von oberflächlichen Läsionen oder Tumoren, insbesondere auf der Haut oder auf der Oberfläche von Organen.

### Bezugszeichenliste

- 10: Strahlentherapievorrichtung
- 11: Strahlungsquelleneinrichtung
- 12: Strahlungsquellenelement
- 13: Tumor

- 20: Applikatoreinrichtung
- 21: Applikatorelement
- 22: Fußbereich
- 23: Führungsbereich
- 24: Aufnahmeraum
- 25: Zweites Ende des Applikatorelements
- 26: Erstes Ende des Applikatorelements
- 27: Element zur Strahlbeeinflussung (Linsenelement)
- 27a: Oberfläche
- 27b: Material
- 27c: Material
- 27d: Oberfläche
- 28: Außendurchmesser

- 30: Befestigungseinrichtung (Befestigungsring)
- 31: Innendurchmesser
- 32: Außenseite
- 33: Befestigungslasche
- 34: Befestigungslasche
- 35: Befestigungsöffnung
- 36: Aufnahmeöffnung

## Patentansprüche

1. Applikatoreinrichtung (20) für die Röntgenstrahlentherapie zur Bestrahlung von Oberflächen, mit einem Applikatorelement (21) zur Aufnahme einer Sondeneinrichtung oder eines Strahlungsquellenelements (12) einer Strahlungsquelleneinrichtung (11), wobei das Applikatorelement (21) zur Einstellung verschiedener Strahlcharakteristika wenigstens ein am/im Applikatorelement (21) angeordnetes Element (27) zur Strahlbeeinflussung aufweist, das Element (27) zur Strahlbeeinflussung als Linsenelement oder als Linsenelementkombination ausgebildet ist, das Linsenelement oder die Linsenelementkombination als Element oder Elementkombination mit einer unterschiedlichen Massenverteilung in einer Ebene quer zur Ausbreitungsrichtung der Röntgenstrahlung ausgebildet ist, das Applikatorelement (21) ein erstes freies Ende (26), das zur Positionierung auf der zu bestrahlenden Oberfläche dient, aufweist, und wobei das Linsenelement oder die Linsenelementkombination im Bereich des ersten freien Endes (26) des Applikatorelements (21) vorgesehen ist, **dadurch gekennzeichnet, dass** das Linsenelement oder die Linsenelementkombination austauschbar ist und an der Außenseite eine ebene Oberfläche aufweist.

2. Applikatoreinrichtung (20) nach Anspruch 1, **dadurch gekennzeichnet, dass** die Massenverteilung des Linsenelements oder der Linsenelementkombination an die Abstrahlcharakteristik einer Strahlungsquelleneinrichtung (11) angepasst oder anpassbar ist.

3. Applikatoreinrichtung (20) nach einem der Ansprüche 1 oder 2, dadurch **gekennzeichnet, dass** das Linsenelement oder die Linsenelementkombination und/oder das Applikatorelement eine runde, oder viereckige, oder achteckige oder an einen Tumor angepasste Form hat.

4. Applikatoreinrichtung (20) nach einem der Ansprüche 1 bis 3, dadurch **gekennzeichnet, dass** das Linsenelement oder die Linsenelementkombination aus einem Material, oder aus wenigstens zwei unterschiedlichen Materialien besteht.

5. Applikatoreinrichtung (20) nach einem der Ansprüche 1 bis 4, dadurch **gekennzeichnet, dass** das Linsenelement oder die Linsenelementkombination wenigstens eine positiv gekrümmte Oberfläche und/oder wenigstens eine neutrale Oberfläche und/oder wenigstens eine negativ gekrümmte Oberfläche aufweist.

6. Applikatoreinrichtung (20) nach einem der Ansprüche 1 bis 5, dadurch **gekennzeichnet, das** das Applikatorelement (21) zumindest bereichsweise zylindrisch ausgebildet ist.

7. Strahlentherapievorrichtung (10) zur Röntgen-Bestrahlung von Oberflächen, mit einer Strahlungsquelleneinrichtung (11) und mit einer Applikatoreinrichtung (20) zur Aufnahme einer Sondeneinrichtung oder eines Strahlungsquellenelements (12) der Strahlungsquelleneinrichtung (11) nach einem der Ansprüche 1 bis 6.

8. Strahlentherapievorrichtung nach Anspruch 7, **dadurch gekennzeichnet, dass** eine Befestigungseinrichtung (30) zur Befestigen der Applikatoreinrichtung (20) auf einer zu behandelnden Oberfläche vorgesehen ist, dass die Befestigungseinrichtung (30) eine Aufnahmeöffnung (36) zur Aufnahme zumindest eines Bereichs der Applikatoreinrichtung (20) aufweist, und dass die Befestigungseinrichtung (30) als Befestigungsring ausgebildet ist.

9. Strahlentherapievorrichtung nach Anspruch 8, **dadurch gekennzeichnet, dass** die Aufnahmeöffnung (36) eine Innenkontur aufweist, die der Außenkontur des aufzunehmenden Bereichs der Applikatoreinrichtung (20) entspricht.

10. Strahlentherapievorrichtung nach Anspruch 8 oder 9, dadurch **gekennzeichnet, dass** die Befestigungseinrichtung (30) an wenigstens einer ihrer Außenseiten (32) wenigstens eine nach außen abragende Befestigungslasche (33, 34) aufweist.

11. Strahlentherapievorrichtung nach Anspruch 10, **dadurch gekennzeichnet, dass** wenigsten eine Befestigungslasche (33, 34) wenigstens eine Befestigungsöffnung (35) aufweist.

## Claims

1. Applicator device (20) for x-ray therapy for irradiating surfaces, comprising an applicator element (21) for receiving a probe device or a radiation source element (12) of a radiation source device (11), wherein the applicator element (21) includes at least one element (27) for influencing the beam, arranged on/in the applicator element (21), for setting various beam characteristics, the element (27) for influencing the beam is embodied as a lens element or as a lens element combination, the lens element or the lens element combination is embodied as an element or element combination with a varying mass distribution in a plane across the propagation direction of the x-ray radiation, the applicator element (21) includes a first free end (26) serving for positioning on the surface to be irradiated and wherein the lens element or the lens element combination is provided in the region of the first free end (26) of the applicator element (21), **characterized in that** the lens element or the lens element combination is replaceable and has a plane surface on the outer side.

2. Applicator device (20) according to Claim 1, **characterized in that** the mass distribution of the lens element or of the lens element combination is adapted or adaptable to the emission characteristic of a radiation source device (11).

3. Applicator device (20) according to either of Claims 1 and 2, **characterized in that** the lens element or the lens element combination and/or the applicator element has a round or quadrilateral or octagonal shape or a shape adapted to a tumour.

4. Applicator device (20) according to one of Claims 1 to 3, **characterized in that** the lens element or the lens element combination consists of one material or of at least two different materials.

5. Applicator device (20) according to one of Claims 1 to 4, **characterized in that** the lens element or the lens element combination includes at least one positively curved surface and/or at least one neutral surface and/or at least one negatively curved surface.

6. Applicator device (20) according to one of Claims 1 to 5, **characterized in that** the applicator element (21) has a cylindrical embodiment, at least in portions.

7. Radiotherapy apparatus (10) for irradiating surfaces with x-rays, comprising a radiation source device (11) and comprising an applicator device (20) for receiving a probe device or a radiation source element (12) of the radiation source apparatus (11) according to one of Claims 1 to 6.

8. Radiotherapy apparatus according to Claim 7, **characterized in that** provision is made for a fastening device (30) for fastening the applicator device (20) onto a surface to be treated, **in that** the fastening device (30) includes a receptacle opening (36) for receiving at least a portion of the applicator device (20) and **in that** the fastening device (30) is embodied as fastening ring.

9. Radiotherapy apparatus according to Claim 8, **characterized in that** the receptacle opening (36) has an inner contour corresponding to the outer contour of the portion of the applicator device (20) to be received.

10. Radiotherapy apparatus according to Claim 8 or 9, **characterized in that** the fastening device (30) includes at least one outwardly projecting fastening lug (33, 34) on at least one of the outer sides (32) thereof.

11. Radiotherapy apparatus according to Claim 10, **characterized in that** at least one fastening lug (33, 34) includes at least one fastening opening (35).

## Revendications

1. Dispositif d'application (20) pour la radiothérapie, permettant d'exposer des surfaces à un rayonnement, pourvu d'un élément d'application (21) destiné à loger un dispositif à sonde ou un élément source de rayonnement (12) d'un dispositif à source de rayonnement (11), l'élément d'application (21), pour le réglage des différentes caractéristiques de faisceau, présentant au moins un élément (27) disposé sur/dans l'élément d'application (21) et destiné à influencer le faisceau, l'élément (27) destiné à influencer le faisceau étant réalisé sous la forme d'un élément lenticulaire ou sous la forme d'une combinaison d'éléments lenticulaires, l'élément lenticulaire ou la combinaison d'éléments lenticulaires étant réalisé(e) sous la forme d'un élément ou d'une combinaison d'éléments présentant une distribution massique différente dans un plan transversal à la direction de propagation du rayonnement X, l'élément d'application (21) comprenant une première extrémité (26), laquelle sert au positionnement sur la surface à exposer au rayonnement, et
l'élément lenticulaire ou la combinaison d'éléments lenticulaires étant disposé(e) dans la zone de la première extrémité libre (26) de l'élément d'application (21), **caractérisé en ce que** l'élément lenticulaire ou la combinaison d'éléments lenticulaires est interchangeable et présente une surface plane sur la face extérieure.

2. Dispositif d'application (20) selon la revendication 1, **caractérisé en ce que** la distribution massique de l'élément lenticulaire ou de la combinaison d'éléments lenticulaires est adaptée ou peut être adaptée à la caractéristique de rayonnement d'un dispositif à source de rayonnement (11).

3. Dispositif d'application (20) selon l'une quelconque des revendications 1 ou 2, **caractérisé en ce que** l'élément lenticulaire ou la combinaison d'éléments lenticulaires et/ou l'élément d'application présentent une forme ronde ou carrée ou octogonale ou adaptée à une tumeur.

4. Dispositif d'application (20) selon l'une quelconque des revendications 1 à 3, **caractérisé en ce que** l'élément lenticulaire ou la combinaison d'éléments lenticulaires est constitué(e) d'un matériau, ou d'au moins deux matériaux différents.

5. Dispositif d'application (20) selon l'une quelconque des revendications 1 à 4, **caractérisé en ce que** l'élément lenticulaire ou la combinaison d'éléments lenticulaires présente au moins une surface courbée de manière positive et/ou au moins une surface neutre et/ou au moins une surface courbée de manière négative.

6. Dispositif d'application (20) selon l'une quelconque des revendications 1 à 5, **caractérisé en ce que** l'élément d'application (21) est cylindrique au moins par endroits.

7. Dispositif de radiothérapie (10) permettant d'exposer des surfaces à un rayonnement, pourvu d'un dispositif à source de rayonnement (11) et d'un dispositif d'application (20) destiné à loger un dispositif à sonde ou un élément source de rayonnement (12) du dispositif à source de rayonnement (11) selon l'une quelconque des revendications 1 à 6.

8. Dispositif de radiothérapie selon la revendication 7, **caractérisé en ce qu'**un dispositif de fixation (30) est conçu pour fixer le dispositif d'application (20) sur une surface à traiter, **en ce que** le dispositif de fixation (30) présente une ouverture de logement (36) destinée à loger au moins une zone du dispositif d'application (20), et **en ce que** le dispositif de fixation (30) est réalisé sous la forme d'une bague de fixation.

9. Dispositif de radiothérapie selon la revendication 8, **caractérisé en ce que** l'ouverture de logement (36) présente un contour intérieur, lequel correspond au contour extérieur de la zone à loger du dispositif d'application (20).

10. Dispositif de radiothérapie selon la revendication 8 ou 9, **caractérisé en ce que** le dispositif de fixation (30) présente sur au moins une de ses faces extérieures (32) au moins une patte de fixation (33, 34) faisant saillie vers l'extérieur.

11. Dispositif de radiothérapie selon la revendication 10, **caractérisé en ce qu'**au moins une patte de fixation (33, 34) présente au moins une ouverture de fixation (35).
